(19) Europäisches Patentamt
European Patent Office
Office européen des brevets

(11) EP 2 093 227 A1

(12) **EUROPEAN PATENT APPLICATION**
published in accordance with Art. 153(4) EPC

(43) Date of publication:
**26.08.2009 Bulletin 2009/35**

(21) Application number: **07849964.7**

(22) Date of filing: **26.11.2007**

(51) Int Cl.:
***C07D 471/22*** (2006.01) ***C09K 11/06*** (2006.01)
***H01L 51/50*** (2006.01) ***C07F 1/08*** (2006.01)

(86) International application number:
**PCT/JP2007/073259**

(87) International publication number:
**WO 2008/066187 (05.06.2008 Gazette 2008/23)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **27.11.2006 JP 2006318299**
**27.11.2006 JP 2006318300**
**31.05.2007 JP 2007144633**

(71) Applicant: **Sumitomo Chemical Company, Limited**
**Tokyo 104-8260 (JP)**

(72) Inventors:
- **SUGIYAMA, Hiroyasu**
  **Tsukuba-shi**
  **Ibaraki 305-0031 (JP)**
- **HIGASHIMURA, Hideyuki**
  **Tsukuba-shi**
  **Ibaraki 305-0045 (JP)**
- **AKINO, Nobuhiko**
  **Koshigaya-shi**
  **Saitama 343-0807 (JP)**

(74) Representative: **Duckworth, Timothy John**
**J.A. Kemp & Co.,**
**14 South Square,**
**Gray's Inn**
**London WC1R 5JJ (GB)**

(54) **POLYNUCLEAR COMPLEX**

(57) A polynuclear complex having two or more metal atoms and/or metal ions per one ligand of the general formula (I):

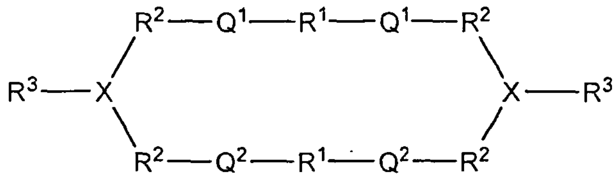

(I)

(wherein, $Q^1$ and $Q^2$ represent each independently a divalent heterocyclic group optionally having a substituent. $R^1$ and $R^2$ represent each independently a direct bond or divalent hydrocarbon group, X represents a nitrogen atom or phosphorus atom, and $R^3$ represents a monovalent organic group containing an atom selected from a nitrogen atom, oxygen atom, phosphorus atom and sulfur atom, or a hydrogen atom or hydrocarbon group

EP 2 093 227 A1

**Description**

TECHNICAL FIELD

The present invention relates to a polynuclear complex.

BACKGROUND ART

Polynuclear metal complexes are paid to attention because of particular functions not observed in mononuclear metal complexes (see, e.g., non-patent document 1). As these functions, for example, a light emitting property, catalytic activity and the like are known.

[non-patent document 1] Chem. Rev. 96, 759 to 833 (1996)

However, the polynuclear structure has a problem that formation of the structure is in general more difficult as compared with a mononuclear structure and the designed molecule cannot necessarily actually be synthesized universally. Namely, there is required a polynuclear complex of which polynuclear structure can be formed relatively easily and having functions such as a light emitting property, catalytic activity or the like.

DISCLOSURE OF THE INVENTION

The present inventors have intensively studied and resultantly found that a polynuclear complex having a certain kind of macrocyclic ligand satisfies such an object, leading to completion of the present invention.

That is, the present invention provides a polynuclear complex having two or more metal atoms and/or metal ions per one ligand of the general formula (I):

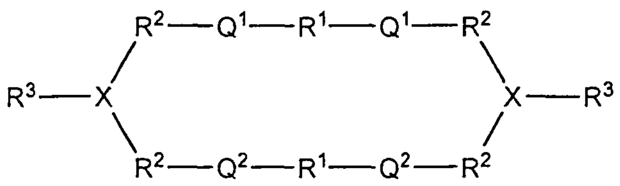

(I)

(wherein, $Q^1$ and $Q^2$ represent each independently a divalent heterocyclic group optionally having a substituent, two $Q^1$s may bond directly or indirectly to form a ring , and two $Q^2$s may bond directly or indirectly to form a ring. $R^1$ and $R^2$ represent each independently a direct bond or an optionally substituted divalent hydrocarbon group, X represents a nitrogen atom or phosphorus atom, $R^3$ represents a monovalent organic group containing an atom selected from a nitrogen atom, oxygen atom, phosphorus atom and sulfur atom, or a hydrogen atom or hydrocarbon group optionally having a substituent, two $R^3$s may bond directly or indirectly to form a ring, and a plurality of $Q^1$s, $Q^2$s, $R^1$s, $R^2$s, $R^3$s and Xs may be mutually the same or different, respectively.).

BRIEF DESCRIPTION OF THE DRAWINGS

Fig. 1 is a graph showing a relation between the modulated frequency of excited light and the modulation, for compound 1.

Fig. 2 is a graph showing a relation between the modulated frequency of excited light and the modulation, for compound 2.

MODES FOR CARRYING OUT THE INVENTION

The polynuclear complex of the present invention is a polynuclear complex having two or more metal atoms and/or metal ions per one ligand of the above-described general formula (I).

In the general formula (I), $Q^1$ and $Q^2$ represent each independently a divalent heterocyclic group optionally having a substituent, a plurality of $Q^1$s and $Q^2$s may be mutually the same or different, respectively, and two $Q^1$s may bond directly or indirectly to form a ring and two $Q^2$s may bond directly or indirectly to form a ring. Here, the divalent heterocyclic group is a group obtained by removing two hydrogen atoms from a heterocyclic compound.

As the heterocyclic compound, preferable are cyclic compounds having a ring member number of 3 to 8 and containing a nitrogen atom, oxygen atom, phosphorus atom and/or sulfur atom and the like in the ring. The ring member number of the hetero ring is preferably 4 to 7, more preferably 5 or 6, further preferably 6. In the hetero ring, a nitrogen atom, oxygen atom, phosphorus atom and/or sulfur atom is preferably contained, a nitrogen atom, oxygen atom and/or phosphorus atom is more preferably contained, a nitrogen atom and/or phosphorus atom is further preferably contained, and a nitrogen atom is particularly preferably contained.

Of the heterocyclic compounds, heterocyclic compounds having an aromatic property are preferable.

Specific examples of the heterocyclic compound include pyrrole optionally having a substituent, furan optionally having a substituent, phosphole optionally having a substituent, thiophene optionally having a substituent, pyrazole optionally having a substituent, imidazole optionally having a substituent, oxazole optionally having a substituent, thiazole optionally having a substituent, triazole optionally having a substituent, thiadiazole optionally having a substituent, oxadiazole optionally having a substituent, pyridine optionally having a substituent, pyrazine optionally having a substituent, pyrimidine optionally having a substituent and triazine optionally having a substituent. Preferable are pyrrole optionally having a substituent, furan optionally having a substituent, phosphole optionally having a substituent, thiophene optionally having a substituent and pyridine optionally having a substituent, more preferable are pyrrole optionally having a substituent and pyridine optionally having a substituent, and particular preferable is pyridine optionally having a substituent.

Specific examples of the divalent heterocyclic group include a pyrrole-2,5-diyl group, furan-2,5-diyl group, phosphole-2,5-diyl group, thiophene-2,5-diyl group, pyrazole-1,3-diyl group, imidazole-2,5-diyl group, oxazole-2,5-diyl group, thiazole-2,5-diyl group, triazole-1,3-diyl group, thiadiazole-2,5-diyl group, oxadiazole-2,5-diyl group, pyridine-2,6-diyl group, pyrazine-2,6-diyl group, pyrimidine-2,6-diyl group and triazine-2,6-diyl group, preferably a pyrrole-2,5-diyl group, furan-2,5-diyl group, phosphole-2,5-diyl group, thiophene-2,5-diyl group and pyridine-2,6-diyl group, more preferably a pyridine-2,6-diyl group and pyrrole-2,5-diyl group, and particularly preferably a pyridine-2,6-diyl group.

When the divalent heterocyclic group has a substituent, the substituent includes a halogen atom, hydroxyl group, mercapto group, amino group, phosphino group, nitro group, cyano group, hydrocarbon group, hydrocarbonoxy group, hydrocarbon mercapto group, hydrocarbon amino group, hydrocarbon phosphino group and the like.

The halogen atom includes a fluorine atom, chlorine atom, bromine atom and iodine atom, preferably a fluorine atom and chlorine atom, more preferably a fluorine atom.

Specific examples of the hydrocarbon group include alkyl groups having 1 to 20 carbon atoms such as a methyl group, ethyl group, propyl group, isopropyl group, butyl group, isobutyl group, t-butyl group, pentyl group, hexyl group, nonyl group, dodecyl group, pentadecyl group, octadecyl group, docosyl group and the like; cycloalkyl groups having 3 to 20 carbon atoms such as a cyclopropyl group, cyclobutyl group, cyclopentyl group, cyclohexyl group, cyclononyl group, cyclododecyl group, norbornyl group, adamantyl group and the like; alkenyl groups having 2 to 20 carbon atoms such as an ethenyl group, propenyl group, 3-butenyl group, 2-butenyl group, 2-pentenyl group, 2-hexenyl group, 2-nonenyl group, 2-dodecenyl group and the like; aryl groups having 6 to 20 carbon atoms such as a phenyl group, 1-naphthyl group, 2-naphthyl group, 2-methylphenyl group, 3-methylphenyl group, 4-methylphenyl group, 4-ethylphenyl group, 4-propylphenyl group, 4-isopropylphenyl group, 4-butylphenyl group, 4-t-butylphenyl group, 4-hexylphenyl group, 4-cyclohexylphenyl group, 4-adamantylphenyl group, 4-phenylphenyl group and the like; aralkyl groups having 7 to 20 carbon atoms such as a phenylmethyl group, 1-phenyleneethyl group, 2-phenylethyl group, 1-phenyl-1-propyl group, 1-phenyl-2-propyl group, 2-phenyl-2-propyl group, 1-phenyl-3-propyl group, 1-phenyl-4-butyl group, 1-phenyl-5-pentyl group, 1-phenyl-6-hexyl group and the like. A the hydrocarbon group, preferable are alkyl groups, aryl groups and aralkyl groups, more preferable are alkyl groups and aryl groups, more preferable are alkyl groups, and particularly preferable are alkyl groups having 1 to 4 carbon atoms.

The hydrocarbonoxy group and hydrocarbon mercapto group are groups obtained by substitution on a hydroxyl group and mercapto group with the above-described hydrocarbon group, respectively.

The hydrocarbon amino group and hydrocarbon phosphino group are groups obtained by substitution on an amino group and phosphino group with one or two of the above-described hydrocarbon groups, respectively.

When the divalent heterocyclic group has a substituent, the substituent includes preferably a halogen atom, nitro group, cyano group, hydrocarbon group, hydrocarbonoxy group, hydrocarbon mercapto group, hydrocarbon amino group and hydrocarbon phosphino group, more preferably a halogen atom, nitro group, cyano group, hydrocarbon group and hydrocarbonoxy group.

As $Q^1$, preferable are divalent heterocyclic groups represented by

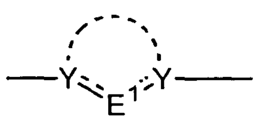

(wherein, $E^1$ represents a nitrogen atom, phosphorus atom, oxygen atom or sulfur atom, and Y represents a carbon atom or nitrogen atom)(the heterocyclic group optionally has a substituent), more preferable are divalent heterocyclic groups represented by

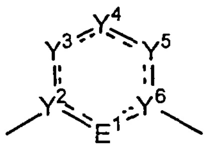

(wherein, $E^1$ represents the same meaning as described above. $Y^2$ and $Y^6$ represent each independently a carbon atom or nitrogen atom, $Y^3$ and $Y^5$ represent each independently C(H), nitrogen atom, N(H), oxygen atom or sulfur atom, and $Y^4$ represents a direct bond, C(H), nitrogen atom, oxygen atom or sulfur atom)(the heterocyclic group optionally has a substituent), and further preferable are divalent heterocyclic groups represented by

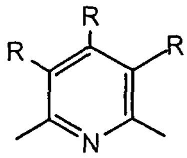

(wherein, R represents a hydrogen atom or substituent, two Rs together may form a ring)(the heteroyclic group optionally has a substituent) or divalent heterocyclic groups represented by

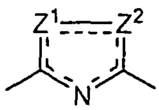

(wherein, either $Z^1$ or $Z^2$ is C(R') and another is an oxygen atom, sulfur atom or N(R''), R' and R'' represent a hydrogen atom or substituent, and two R's or two R'' s together may form a ring)(the heterocyclic group optionally has a substituent).

As $Q^2$, preferable are divalent heterocyclic groups represented by

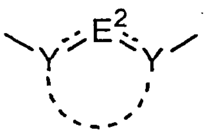

(wherein, $E^2$ represents a nitrogen atom, phosphorus atom, oxygen atom or sulfur atom, and Y represents a carbon atom or nitrogen atom)(the heterocyclic group optionally has a substituent), more preferable are divalent heterocyclic groups represented by

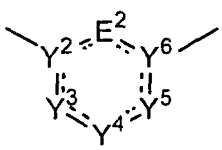

(wherein, $E^2$ represents the same meaning as described above. $Y^2$ and $Y^6$ represent each independently a carbon atom or nitrogen atom, $Y^3$ and $Y^5$ represent each independently C(H), nitrogen atom, N(H), oxygen atom or sulfur atom, and $Y^4$ represents a direct bond, C(H), nitrogen atom, oxygen atom or sulfur atom)(the heterocyclic group optionally has a substituent), and further preferable are divalent heterocyclic groups represented by

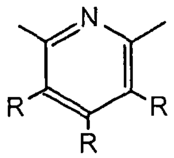

(wherein, R represents a hydrogen atom or substituent, and two Rs together may form a ring) or by

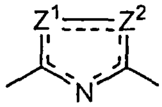

(wherein, either $Z^1$ or $Z^2$ is C(R') and another is a oxygen atom, sulfur atom or N(R"), R' and R" represent a hydrogen atom or substituent, and two R's or two R"s together may form a ring)(the heterocyclic group optionally has a substituent).

$R^1$ and $R^2$ in the above-described general formula (I) represent each independently a direct bond or optionally substituted divalent hydrocarbon group, and a plurality of $R^1$s and $R^2$s may be mutually the same or different, respectively.

The divalent hydrocarbon group includes alkylene groups having 1 to 20 carbon atoms such as a methylene group, ethane-1,1-diyl group, ethane-1,2-diyl group, propane-1,1-diyl group, propane-1,2-diyl group, propane-1,3-diyl group, propane-2,2-diyl group, butane-1,1-diyl group, butane-1,2-diyl group, butane-1,3-diyl group, butane-1,4-diyl group, butane-2,2-diyl group, butane-2,3-diyl group, pentane-1,1-diyl group, pentane-1,2-diyl group, pentane-1,5-diyl group, hexane-1,1-diyl group, hexane-1,2-diyl group, hexane-1,6-diyl group, nonane-1,1-diyl group, nonane-1,2-diyl group, nonane-1,9-diyl group, dodecane-1,1-diyl group, dodecane-1,2-diyl group, dodecane-1,12-diyl group and the like; cycloalkylene groups having 3 to 20 carbon atoms such as a cyclopropane-1,1-diyl group, cyclopropane-1,2-diyl group, cyclobutane-1,1-diyl group, cyclobutane-1,2-diyl group, cyclobutane-1,3-diyl group, cyclopentane-1,1-diyl group, cyclopentane-1,2-diyl group, cyclopentane-1,3-diyl group, cyclononane-1,1-diyl group, cyclononane-1,2-diyl group, cyclononane-1,3-diyl group, cyclododecane-1,1-diyl group, cyclododecane-1,2-diyl group, cyclododecane-1,3-diyl group and the like; alkenylene groups having 2 to 20 carbon atoms such as an ethene-1,1-diyl group, ethene-1,2-diyl group, propene-1,1-diyl group, propene-1,2-diyl group, propene-1,3-diyl group, propene-2,2-diyl group, 1-butene-1,1-diyl group, 1-butene-1,2-diyl group, 1-butene-1,3-diyl group, 1-butene-1,4-diyl group, 1-butene-2,2-diyl group, 1-butene-2,3-diyl group, 2-butene-1,1-diyl group, 2-butene-1,2-diyl group, 2-butene-1,3-diyl group, 2-butene-1,4-diyl group, 2-butene-2,3-diyl group, 1-pentene-1,1-diyl group, 1-pentene-1,2-diyl group, 1-pentene-1,5-diyl group, 1-nonene-1,1-diyl group, 1-nonene-1,2-diyl group, 1-nonene-1,9-diyl group, 1-dodecene-1,1-diyl group, 1-dodecene-1,2-diyl group, 1-dodecene-1,12-diyl group and the like; alkynylene groups having 2 to 20 carbon atoms such as an ethyne-1,2-diyl group, propyne-1,3-diyl group, 1-butyne-1,3-diyl group, 1-butyne-1,4-diyl group, 2-butyne-1,4-diyl group, 1-pentyne-1,3-diyl group, 1-pentyne-1,4-diyl group, 1-pentyne-1,5-diyl group, 2-pentyne-1,4-diyl group, 2-pentyne-1,5-diyl group, 1-nonyne-1,3-diyl group, 1-nonyne-1,9-diyl group, 1-dodecyne-1,3-diyl group, 1-dodecyne-1,12-diyl group and the like; arylene groups having 6 to 20 carbon atoms such as a 1,2-phenylene group, 1,3-phenylene group, 1,4-phenylene group, 1,2-naphthylene group, 1,4-naphthylene group, 1,5-naphthylene group, 2,3-naphthylene group, 2,6-naphthylene group, 3-phenyl-1,2-phenylene group, 2,2'-diphenylene group and the like; divalent hydrocarbon groups having 7 to 20 carbon atoms composed of arylene groups and alkylene groups, such as a 1,2-phenylenemethylene group, 1,3-phenylenemethylene group, 1,4-phenylenemethylene group, 1,2-phenylene-1,1-ethylene group, 1,2-phenylene-1,2-ethylene group, 1,2-phenylene-1,2-propylene group, 1,2-phenylene-1,3-propylene group, 1,2-phenylene-1,4-butylene group, 1,2-phenylene-1,2-butylene group, 1,2-phenylene-1,2-hexylene group, methylene-1,2-phenylenemethylene group, methylene-1,3-phenylenemethylene group, methylene-1,4-phenylenemethylene group and the like.

When the divalent hydrocarbon group has a substituent, specific examples and preferable examples of the substituent are the same as those in the explanation of the substituent optionally carried on the divalent heterocyclic group.

$R^1$ represents preferably a direct bond, alkylene group having 1 to 8 carbon atoms, cycloalkylene group, alkenylene group, arylene group, or divalent hydrocarbon group composed of an arylene group and an arylenealkylene group, more preferably a direct bond, alkylene group having 1 to 6 carbon atoms, alkenylene group or arylene group, further preferably a direct bond, methylene group, ethane-1,2-diyl group, propane-1,3-diyl group, ethene-1,2-diyl group or 1,2-phenylene group, and particularly preferably a direct bond.

$R^2$ represents preferably a direct bond, alkylene group having 1 to 8 carbon atoms, cycloalkylene group,

alkenylene group, arylene group, or divalent hydrocarbon group composed of an arylene group and an arylenealkylene group, more preferably an alkylene group having 1 to 6 carbon atoms or arylene group, further preferably a methylene group, ethane-1,2-diyl group, propane-1,3-diyl group or 1,2-phenylene group, and particularly preferably a methylene group.

In the general formula (I), two $Q^1$s may bond directly or indirectly to form a ring, and two $Q^2$s may bond directly or indirectly to form a ring. In this case, specific examples of the divalent group represented by $-Q^1-R^1-Q^1-$ and the divalent group represented by $-Q^2-R^1-Q^2-$ in the above-described formula (I) include a 1,10-phenanthroline-2,9-diyl group, 1,10-phenanthroline-3,8-diyl group, 4,5-diazafluorene-3,6-diyl group, 4,5-diazafluorene-2,7-diyl group and the like. Preferable are a 1,10-phenanthroline-2,9-diyl group and 4,5-diazafluorene-3,6-diyl group, more preferable is a 1,10-phenanthroline-2,9-diyl group.

X in the above-described general formula (I) represents a nitrogen atom or phosphorus atom, and two Xs may be the same or different.

X represents preferably a nitrogen atom.

$R^3$ in the above-described general formula (I) represents a hydrogen atom, a hydrocarbon group optionally having a substituent, or a monovalent organic group containing an atom selected from a nitrogen atom, oxygen atom, phosphorus atom and sulfur atom, and two $R^3$s may bond directly or indirectly to form a ring and two $R^3$s may be the same or different.

Specific examples and preferable examples of the hydrocarbon group represented by $R^3$ are the same as those for the above-described hydrocarbon group, and when the hydrocarbon group has a substituent, specific examples and preferable examples of the substituent are the same as those in the explanation of the substituent optically carried on the divalent heterocyclic group.

The monovalent organic group containing an atom selected from a nitrogen atom, oxygen atom, phosphorus atom and sulfur atom represented by $R^3$ includes hydrocarbon amino groups, hydrocarbon phosphino groups, hydrocarbon mercapto groups, and groups obtained by bonding a divalent organic group to a group prepared by removing one hydrogen atom from a heterocyclic compound, or monovalent heterocyclic groups (group obtained by removing one hydrogen atom from heterocyclic compound).

Specific examples and preferable examples of the heterocyclic compound are the same as those in the explanation of the divalent heterocyclic group.

Specific examples of the monovalent organic group containing an atom selected from a nitrogen atom, oxygen atom, phosphorus atom and sulfur atom represented by $R^3$ include monovalent organic groups containing a nitrogen atom such as a dimethylaminomethyl group, diethylaminomethyl group, diisopropylaminomethyl group, diphenylaminomethyl group, dicyclohexylaminomethyl group, dimethylaminoethyl group, diethylaminoethyl group, diisopropylaminoethyl group, diphenylaminoethyl group, dicyclohexylaminoethyl group, dimethylaminophenyl group, diethylaminophenyl group, diisopropylaminophenyl group, diphenylaminophenyl group, dicyclohexylaminophenyl group and the like; monovalent organic groups containing a phosphorus atom such as a dimethylphosphinomethyl group, diethylphosphinomethyl group, diisopropylphosphinomethyl group, diphenylphosphinomethyl group, dicyclohexylphosphinomethyl group, dimethylphosphinoethyl group, diethylphosphinoethyl group, diisopropylphosphinoethyl group, diphenylphosphinoethyl group, dicyclohexylphosphinoethyl group, dimethylphosphinophenyl group, diethylphosphinophenyl group, diisopropylphosphinophenyl group, diphenylphosphinophenyl group, dicyclohexylphosphinophenyl group and the like; monovalent organic groups containing a sulfur atom such as a methylmercaptomethyl group, ethylmercaptomethyl group, isopropylmercaptomethyl group, phenylmercaptomethyl group, cyclohexylmercaptomethyl group, methylmercaptoethyl group, ethylmercaptoethyl group, isopropylmercaptoethyl group, phenylmercaptoethyl group, cyclohexylmercaptoethyl group, methylmercaptophenyl group, ethylmercaptophenyl group, isopropylmercaptophenyl group, phenylmercaptophenyl group, cyclohexylmercaptophenyl group and the like; monovalent organic groups containing a hetero ring such as a 2-pyridylmethyl group, 2-pyrrolylmethyl group, 2-phosphorylmethyl group, 2-furylmethyl group, 2-thienylmethyl group, 2-pyridylethyl group, 2-pyrrolylethyl group, 2-phosphorylethyl group, 2-furylethyl group, 2-thienylethyl group, 2-pyrrolylethyl group, 2-pyridyl group, 2-pyrrolyl group, 2-phosphoryl group, 2-furyl group, 2-thienyl group, 8-quinolyl group, 7-indolyl group, 7-benzofuryl group, 7-benzothienyl group, and the like. The monovalent organic groups containing a nitrogen atom, the monovalent organic groups containing a phosphorus atom and the monovalent organic groups containing a hetero ring are preferable, and a dimethylphosphinomethyl group, diethylphosphinomethyl group, diisopropylphosphinomethyl group, diphenylphosphinomethyl group, dicyclohexylphosphinomethyl group, dimethylphosphinoethyl group, diethylphosphinoethyl group, diisopropylphosphinoethyl group, diphenylphosphinoethyl group, dicyclohexylphosphinoethyl group, dimethylphosphinophenyl group, diethylphosphinophenyl group, diisopropylphosphinophenyl group, diphenylphosphinophenyl group, dicyclohexylphosphinophenyl group, 2-pyridylmethyl group, 2-pyrrolylmethyl group, 8-quinolyl group and 7-indolyl group are more preferable, and a diphenylphosphinophenyl group and 2-pyridylmethyl group are further preferable.

As $R^3$, preferable are a hydrogen atom, alkyl group, aryl group, aralkyl group, monovalent organic groups containing a nitrogen atom, monovalent organic groups containing a phosphorus atom and monovalent organic groups

containing a hetero ring, more preferable are a hydrogen atom, alkyl group, aryl group, dimethylphosphinomethyl group, diethylphosphinomethyl group, diisopropylphosphinomethyl group, diphenylphosphinomethyl group, dicyclohexylphosphinomethyl group, dimethylphosphinoethyl group, diethylphosphinoethyl group, diisopropylphosphinoethyl group, diphenylphosphinoethyl group, dicyclohexylphosphinoethyl group, dimethylphosphinophenyl group, diethylphosphinophenyl group, diisopropylphosphinophenyl group, diphenylphosphinophenyl group, dicyclohexylphosphinophenyl group, 2-pyridylmethyl group, 2-pyrrolylmethyl group, 8-quinolyl group and 7-indolyl group, and further preferable are a hydrogen atom, alkyl groups having 1 to 4 carbon atoms, diphenylphosphinophenyl group and 2-pyridylmethyl group.

Among the ligands of the above-described general formula (I), preferable are ligands of the following general formula (II).

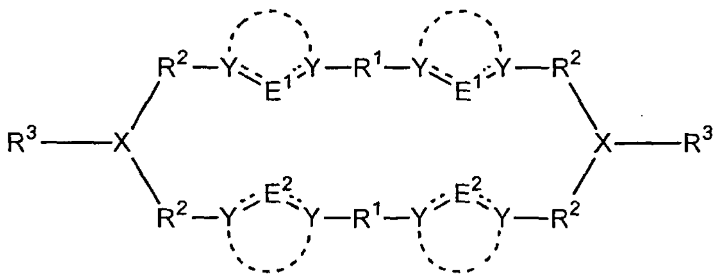

(II)

(wherein, $R^1$, $R^2$ , $R^3$ and X represent each independently the same meaning as described above. $E^1$ and $E^2$ represent each independently a nitrogen atom, phosphorus atom, oxygen atom or sulfur atom, Y represents a carbon atom or nitrogen atom, and a plurality of $E^1$s, $E^2$s, $R^1$s, $R^2$s, $R^3$s, Xs and Ys may be mutually the same or different, respectively. Two $R^3$ may bond directly or indirectly to form a ring, a ring represented by

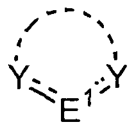

optionally has a substituent,
two rings represented by

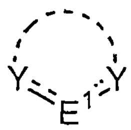

may form a ring directly or together with the substituent on the ring, and a ring represented by

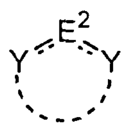

optionally has a substituent,
two rings represented by

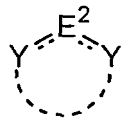

may form a ring directly or together with the substituent on the ring.).

Among the ligands of the above-described general formula (II), preferable are ligands of the following general formula (III):

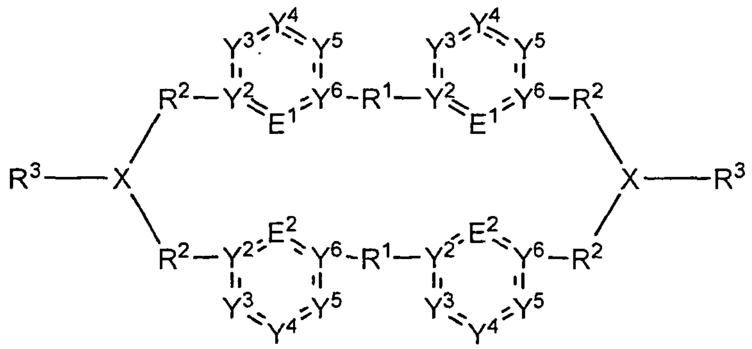

(III)

(wherein, $E^1$, $E^2$, X, $R^1$, $R^2$ and $R^3$ represent each independently the same meaning as described above. $Y^2$ and $Y^6$ represent each independently a carbon atom or nitrogen atom, $Y^3$ and $Y^5$ represent each independently C(H), nitrogen atom, N(H), oxygen atom or sulfur atom, $Y^4$ represents a direct bond, C(H), nitrogen atom, oxygen atom or sulfur atom, and a plurality of $E^1$s, $E^2$s, $R^1$s, $R^2$s, $R^3$s, Xs and $Y^3$s to $Y^5$s may be mutually the same or different, respectively. Two $R^3$ may bond directly or indirectly to form a ring,
a ring represented by

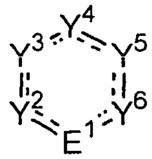

optionally has a substituent,
substituents on two rings represented by

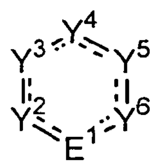

may together form a ring,
and a ring represented by

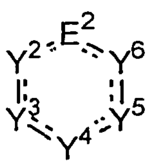

optionally has a substituent,
substituents on two rings represented by

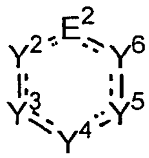

may together form a ring.).

Among the ligands of the above-described general formula (III), preferable are ligands of the following general formulae (IV) and (V):

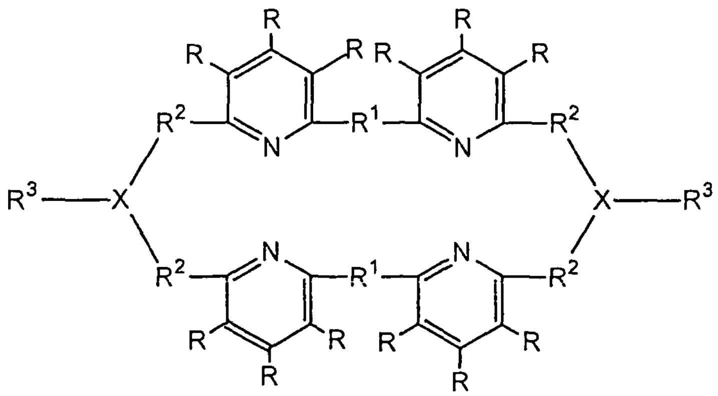

(IV)

(wherein, X, $R^1$, $R^2$ and $R^3$ represent each independently the same meaning as described above. R represents a hydrogen atom or substituent, and two Rs together may form a ring.)

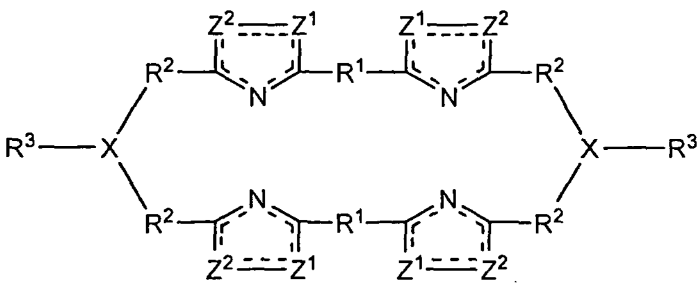

(V)

(wherein, X, $R^1$, $R^2$ and $R^3$ represent each independently the same meaning as described above. Either $Z^1$ or $Z^2$ is C(R') and another is an oxygen atom, sulfur atom or N(R''), $Z^1$s may be mutually the same or different and $Z^2$s may be mutually the same or different. R' and R'' represent each independently a hydrogen atom or substituent, and two R's or R''s together may form a ring.).

Regarding $Z^1$ and $Z^2$, it is preferable that $Z^1$ is C(R') and $Z^2$ is an oxygen atom, sulfur atom or N(R'').

Definitions, specific examples and preferable examples of the substituent for R' and R'' are the same as those in the explanation of the substituent optionally carried on the divalent heterocyclic group.

The metal atom and metal ion carried on the polynuclear complex of the present invention are not particularly restricted providing they are atoms and ions of metal elements, and preferable are atoms and ions of groups I to XII metal elements, more preferable are atoms and ions of groups III to XII metal elements, further preferable are atoms and ions of groups III to XII, fourth period metal elements, and particularly preferable are a copper ion and a silver ion.

As the valency of the metal atom and/or metal ion, those generally present in the natural world may be appropriately selected and used, and for example, in the case of copper, monovalency or divalency, or a mixed atomic valency containing both of them, may be permissible.

In the polynuclear complex of the present invention, the number of the metal atoms is two or more per one ligand compound of the general formula (I). When the number of the metal atom is one per one ligand compound of the general formula (I), functions and catalytic performance are not manifested sufficiently.

The number of the metal atoms is preferably 2 to 6, more preferably 2 to 4, further preferably 2 or 3, and particularly preferably 2 per one ligand compound of the general formula (I).

The number of d electrons of the metal atom and/or metal ion is preferably an even number, more preferably 6, 8 or 10, further preferably 10.

Especially, the metal ion is preferably a copper(I) ion or silver(I) ion, particularly preferably a copper(I) ion.

In the polynuclear complex of the present invention, a counter ion for keeping electric neutrality is necessary in some cases. As the counter anion, conjugated bases of Broenstead acids are usually used. Examples thereof include a fluoride ion, chloride ion, bromide ion, iodide ion, sulfate ion, nitrate ion, carbonate ion, acetate ion, perchlorate ion, tetrafluoroborate ion, hexafluorophosphate ion, methanesulfonate ion, trifluoromethanesulfonate ion, trifluoroacetate ion, benzenesulfonate ion, p-toluenesulfonate ion, dodecylbenzenesulfonate ion, tetraphenylborate ion, tetrakis(pentafluorophenyl)borate ion, polymer compounds containing a repeating unit having the structure of these ions, and the like. Preferable are a chloride ion, bromide ion, iodide ion, sulfate ion, nitrate ion, tetrafluoroborate ion, hexafluorophosphate ion, trifluoromethanesulfonate ion, tetraphenylborate ion and tetrakis(pentafluorophenyl)borate ion. As the counter cation, there can be used metal cations of alkali metals, alkaline earth metals and the like, quaternary ammonium ion, quaternary phosphonium ion, polymer compounds having a repeating unit having the structure of these ions, and the like, and preferable are a quaternary ammonium ion and quaternary phosphonium ion.

In the polynuclear complex of the present invention, the structure of parts other than the metal atom and the ligand of the above-described general formula (I) is not particularly restricted, and an additional ligand and the like may be coordinated on the metal.

The additional ligand may be a solvent molecule used in production of a complex.

Examples of the additional ligand include aliphatic nitriles such as acetonitrile, propionitrile, pivalonitrile and the like; aromatic nitriles such as benzonitrile, 2-naphthonitrile, 9-anthracenecarbonitrile and the like; pyridines such as pyridine, picoline, 4-t-butylpyridine, 4-dimethylaminopyridine, quinoline, isoquinoline and the like; amines such as trimethylamine, triethylamine, triphenylamine, tricyclohexylamine and the like; aliphatic phosphines such as trimethylphosphine, triethylphosphine, tricyclohexylphosphine and the like; aromatic phosphines such as dimethylphenylphosphine, diphenylmethylphosphine, triphenylphosphine, tri(p-fluorophenyl)phosphine, tri(p-tolylphosphine), tri(p-methoxyphenyl) phosphine, tri(2-naphthyl)phosphine and the like; aromatic phosphites such as triphenylphosphite, tri(p-tolylphosphite), tri(2-naphthyl)phosphate, and the like. Preferable are aliphatic nitriles, aromatic nitriles, aliphatic phosphines, aromatic phosphines and aromatic phosphites, more preferable are aromatic nitriles, aromatic phosphines and aromatic phosphites, further preferable are aromatic phosphines, and particularly preferable is tri(p-fluorophenyl)phosphine).

The polynuclear complex of the present invention includes, specifically, the following compounds.

$$(L^1)\ (M)\ x\ (L^2)\ y\ (A)\ z$$

$L^1$: ligands of the general formula (I), (II), (III) or (IV) M: metal atoms or metal ions described each independently above
$L^2$: additional ligand described above
A: counter ion described above
x, y, z: integer of 2 to 6

More specifically, examples of the preferable polynuclear complex in the present invention include those of the general formula (Ia).

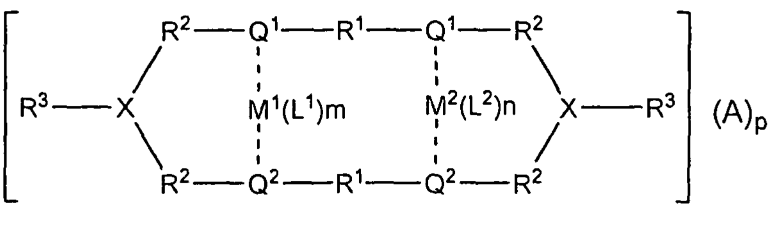

(Ia)

(wherein, $Q^1$ and $Q^2$ represent each independently a divalent heterocyclic group optionally having a substituent, two $Q^1$s may bond directly or indirectly to form a ring, and two $Q^2$s may bond directly or indirectly to form a ring. $R^1$ and

$R^2$ represent each independently a direct bond or a divalent hydrocarbon group optionally having a substituent, X represents a nitrogen atom or phosphorus atom, $R^3$ represents a hydrogen atom, a hydrocarbon group optionally having a substituent, or a monovalent organic group containing an atom selected from a nitrogen atom, oxygen atom, phosphorus atom and sulfur atom, two $R^3$s may bond directly or indirectly to form a ring, and a plurality of $Q^1$s, $Q^2$s, $R^1$s, $R^2$s, $R^3$s and Xs may be mutually the same or different, respectively. $M^1$ and $M^2$ represent each independently a metal atom or metal ion, $L^1$ and $L^2$ represent an additional ligand which can be coordinated on $M^1$ and $M^2$, respectively, m and n represent each independently an integer of 1 to 4, and when there exist a plurality of $L^1$s and $L^2$s, respectively, these may be the same or different, A represents a counter ion, and p represents a number for attaining the electric neutrality of the compound of the structural formula (Ia). When there exist a plurality of As, these may be the same or different.).

A dashed line connecting $Q^1$ and $M^1$, a dashed line connecting $Q^2$ and $M^1$, a dashed line connecting $Q^1$ and $M^2$, and a dashed line connecting $Q^2$ and $M^2$ in the above-described formula (Ia) represent coordinate bonds respectively.

Among the polynuclear complexes of the above-described general formula (Ia), preferable are polynuclear complexes of the following general formula (IIa).

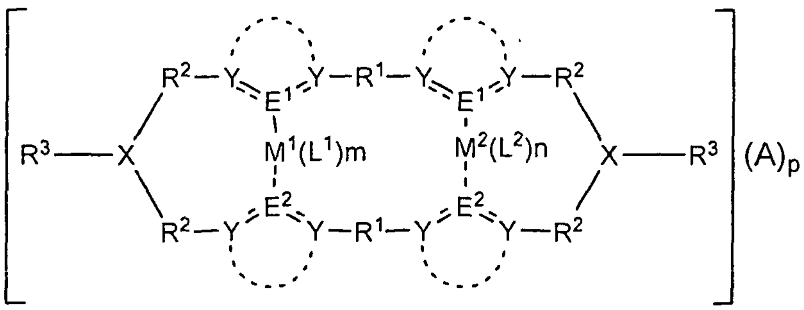

(IIa)

(wherein, $R^1$, $R^2$, $R^3$, $M^1$, $M^2$, $L^1$, $L^2$, m, n, A, p and X represent each independently the same meaning as described above. $E^1$ and $E^2$ represent each independently a nitrogen atom, phosphorus atom, oxygen atom or sulfur atom, Y represents a carbon atom or nitrogen atom, and a plurality of $E^1$s, $E^2$s, $R^1$s, $R^2$s, $R^3$s, Xs and Ys may be mutually the same or different. When there exist a plurality of As, these may be the same or different. Two $R^3$s may bond directly or indirectly to form a ring,

a ring represented by

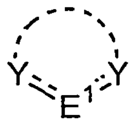

optionally has a substituent,

two rings represented by

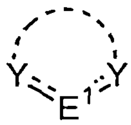

may form a ring directly or together with the substituent on the ring,

and a ring represented by

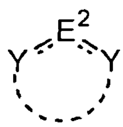

optionally has a substituent,
two rings represented by

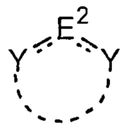

may form a ring directly or together with the substituent on the ring.).

Among the polynuclear complexes of the above-described general formula (IIa), preferable are polynuclear complexes of the following general formula (IIIa).

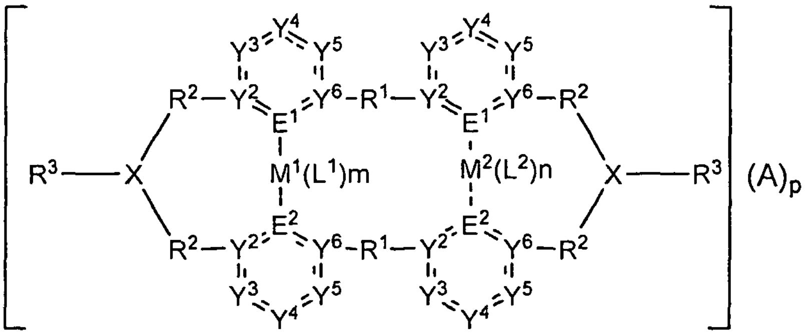

(IIIa)

(wherein, $E^1$, $E^2$, X, $R^1$, $R^2$, $R^3$, A, $M^1$, $M^2$, $L^1$, $L^2$, m, n and p represent each independently the same meaning as described above. $Y^3$ and $Y^5$ represent each independently C(H), nitrogen atom, N(H), oxygen atom or sulfur atom, $Y^4$ represents a direct bond, C(H), nitrogen atom, oxygen atom or sulfur atom, a plurality of $E^1$s, $E^2$s, $R^1$s, $R^2$s, $R^3$s, Xs and $Y^3$s to $Y^5$s may be mutually the same or different, respectively. When there exist a plurality of As, these may be the same or different. Two $R^3$s may bond directly or indirectly to form a ring,
a ring represented by

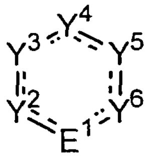

optionally has a substituent,
two rings represented by

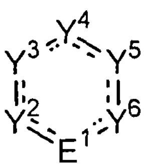

may form a ring directly or together with the substituent on the ring,
and a ring represented by

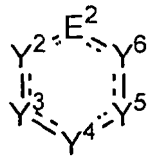

optionally has a substituent,
two rings represented by

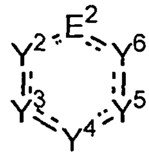

may form a ring directly or together with the substituent on the ring.).

A dashed line connecting $E^1$ and $M^1$, a dashed line connecting $E^2$ and $M^1$, a dashed line connecting $E^1$ and $M^2$, and a dashed line connecting $E^2$ and $M^2$ in the above-described formulae (IIa) and (IIIa) represent coordinate bonds respectively.

Among the polynuclear complexes of the above-described general formula (IIIa), preferable are polynuclear complexes of the following general formula (IVa) or (Va). From the standpoint of light emitting property, the following general formula (IVa) is more preferable.

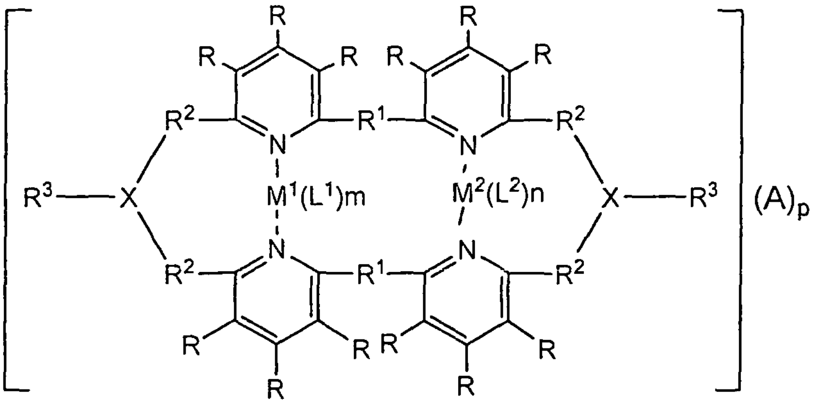

(IVa)

(wherein, X, $R^1$, $R^2$, $R^3$, R, A, $M^1$, $M^2$, $L^1$, $L^2$, m, n and p and $R^3$ represent each independently the same meaning as described above. When there exist a plurality of As, these may be the same or different.).

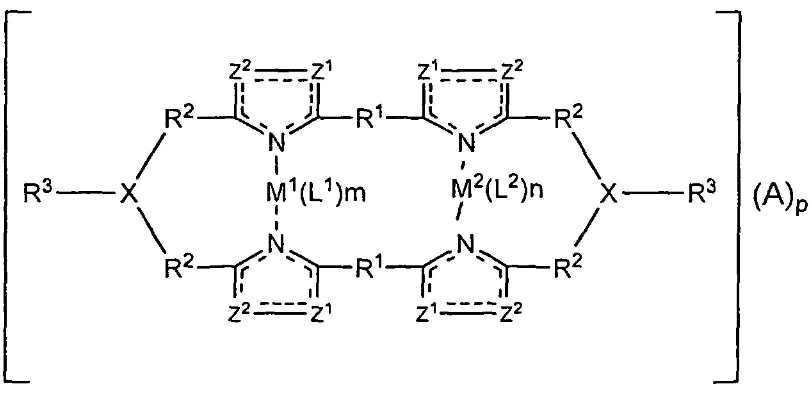

(Va)

(wherein, X, $R^1$, $R^2$, $R^3$, $Z^1$, $Z^2$, A, $M^1$, $M^2$, $L^1$, $L^2$, m, n and p and $R^3$ represent each independently the same meaning as described above. When there exist a plurality of As, these may be the same or different.).

A bond of N and $M^1$ and a bond of N and $M^2$ in the above-described formulae (IVa) and (Va) represent coordinate bonds respectively.

As the polynuclear complex of the present invention, the following complexes are mentioned specifically.

(counter ion is abbreviated in the following formulae)

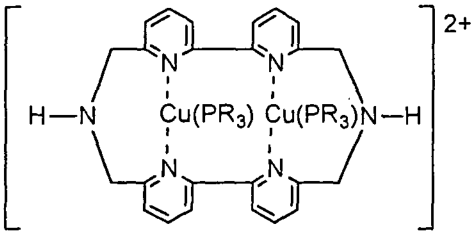

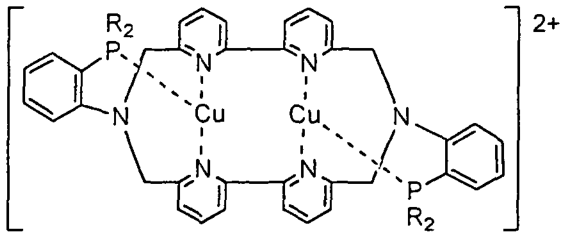

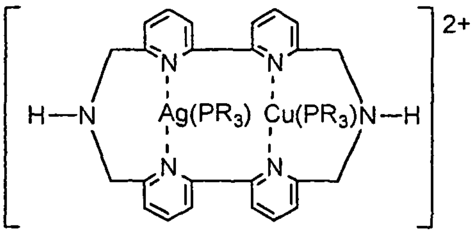

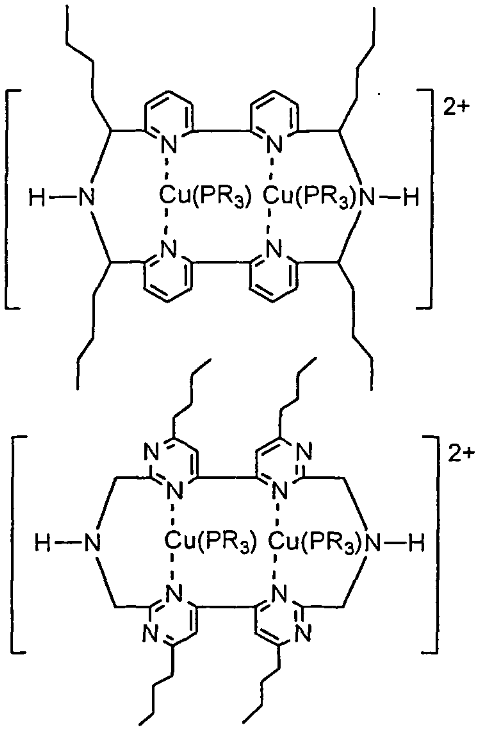

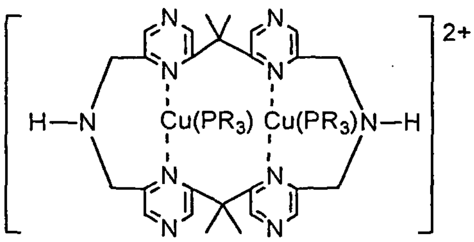

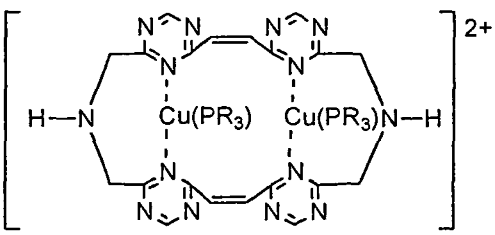

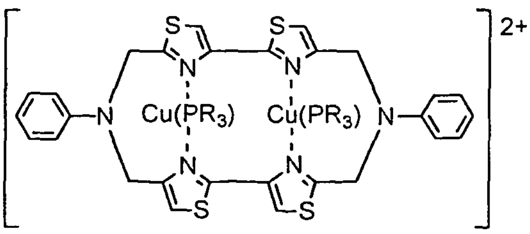

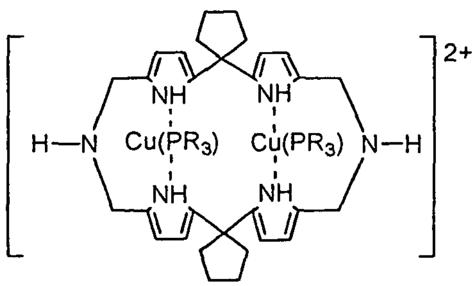

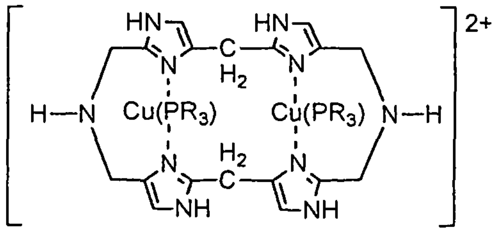

The polynuclear complex of the present invention can be synthesized as described below.

As the ligand compound of the general formula (I), compounds (A) and compounds (B) are preferable typical examples, and these can be synthesized as described in Helv. Chim. Acta., 67, 2264-2269 (1984). The compound (A) or (B) can react with a salt of a metal which should be a center metal in a suitable solvent, to obtain a polynuclear complex thereof.

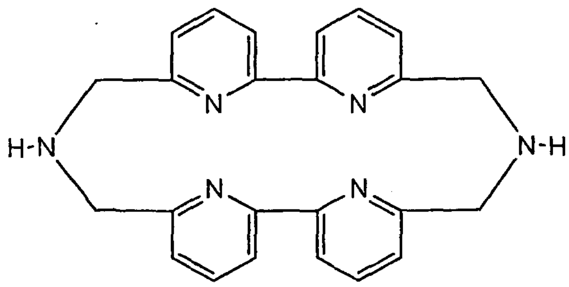

(A)

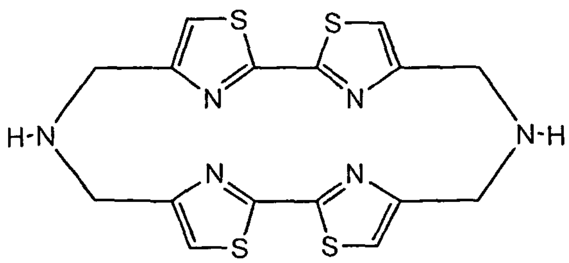

(B)

The polynuclear complex of the present invention preferably has a phosphorescence emitting property and/or fluorescence emitting property, and those having a phosphorescence emitting property are preferable from the standpoint of light emission efficiency.

The polynuclear complex of the present invention can be used in known applications of the polynuclear complex by appropriately selecting its structure. Specifically mentioned are photoelectric-related materials such as light emitting materials, light wavelength conversion materials, light generation materials and the like; catalysts for a redox reaction, organic synthesis reaction, polymer synthesis reaction and the like; magnetic materials and the like. Preferable are photoelectric-related materials such as light emitting materials, light wavelength conversion materials, light generation materials and the like.

The luminous film of the present invention contains a polynuclear complex of the present invention, and those containing a polynuclear complex of the present invention and a polymer are preferable.

The polymer to be used in the luminous film is not particularly restricted, and known polymers can be appropriately selected and used, and those which are soluble in a solvent and are stable are preferable.

Especially, polymers used as a host material of the luminous film are preferably used owing to stability and carrier transportation. Specific examples of such polymers include polymethyl methacrylate, polymethacrylic acid , polymethyl acrylate, polyacrylic acid, polyethylene, polypropylene, polyvinyl ether, polyvinyl chloride, poly vinylidene chloride, polyvinylidene fluoride, polyacrylonitrile, polymethacrylonitrile, polycarbonate, polystyrene, polyvinylcarbazole, polyphenylene, poly-p-phenylenevinylene, poly-p-alkoxy phenylenevinylene, polyfluorene, polybenzfluorene, polyvinyl acetate, polybutadiene, polyisoprene, polychloroprene, polyisobutylene, polyacetylene, polythiophene, polypyrrole, polynorbornene, polysiloxane, polyoxymethylene, polyoxyethylene, polyoxypropylene, polyoxybutylene, polyoxyphenylene, polyurethane, polyethylene terephthalate, poly(1,4-phenylenephenylimino-1,4-phenylene), and copolymers and derivatives thereof. From the standpoint of carrier transportation, so-called conjugated polymers are preferable, and examples thereof include polyphenylene, poly-p-phenylenevinylene, poly-p-alkoxyphenylenevinylene, polyfluorene, polybenzfluorene, polyacetylene, polythiophene, polypyrrole, and the like.

The amount of a polynuclear complex in the luminous film is usually 0.001 to 100 wt%, preferably 0.01 to 98 wt%, more preferably 0.1 to 95 wt% with respect to the total weight of the polynuclear complex and polymer.

The thickness of the luminous film is usually about 100 nm to 100 μm, preferably 100 nm to 1 μm.

In the luminous film of the present invention, a polynuclear complex may be dispersed uniformly in the film, or a part of a polynuclear complex may be present in the form of particle in the film.

When a part of a polynuclear complex is present in the form of particle in the film, if the size of the particle is too large, the luminous film itself cannot be formed uniformly, or irregularity on its surface tends to be remarkable. Therefore, it is preferable that the size of the particle is smaller than the thickness of the luminous film. Further specifically, the size can be usually in the range of 0.1 μm to 10 μm, preferably 0.1 μm to 1 μm, further preferably 0.1 μm to 0.5 μm. Though the shape of the polynuclear complex particle is not particularly restricted, it is not necessary that all sides have the same size, and needle or plate shape may be permissible. When the shape of the polynuclear complex particle is needle or plate, it is preferable that the particles are oriented so as to cause light emission toward a direction vertical to the film surface. As the method for measurement of the size of the particle, known methods for measuring particles can be appropriately used, and for example, observation by electron microscope and the like can be used.

As the method for producing a luminous film of the present invention, for example, a method is mentioned including a step of applying a liquid containing a polynuclear complex, polymer and solvent. The polynuclear complex may be dissolved or dispersed in the form of particle (for example, fine particle, colloid and the like) in a liquid, and it is preferable that a polymer is not dispersed but dissolved.

Specific examples of the solvent include alcohols (methanol, ethanol, isopropyl alcohol, and the like), ketones (acetone, methyl ethyl ketone, and the like), organic chlorine compounds (chloroform, 1,2-dichloroethane and the like), aromatic hydrocarbons (benzene, toluene, xylene, and the like), aliphatic hydrocarbons (normal hexane, cyclohexane and the like), amides (dimethylformamide, and the like), sulfoxides (dimethyl sulfoxide and the like), etc. The solvent may be composed of a single component or a mixture of several components.

As the application method, known methods can be appropriately selected and used. Examples of such methods include casting, spin coat, dip coat, gravure coat, bar coat, roll coat, spray coat, screen printing, flexo printing, offset printing and the like. The film of the present invention can be obtained by removing a solvent after application, and depending on the boiling point of the solvent, it is possible to perform heating to accelerate the removal, thereby reducing the residual solvent.

The light emitting device of the present invention contains a polynuclear complex of the present invention.

As the light emitting device of the present invention, there are mentioned light emitting devices having at least one light emitting layer between a pair of electrodes composed of an anode and a cathode wherein the light emitting layer contains a polynuclear complex of the present invention.

The content of a polynuclear complex of the present invention in the above-described light emitting layer is usually 0.001 to 100 wt%, preferably 0.01 to 98 wt%, more preferably 0.1 to 95 wt% with respect to the weight of the whole light emitting layer. In the light emitting device of the present invention, it is preferable that the above-described light emitting layer contains a polynuclear complex of the present invention as the light emitting material.

The light emitting device of the present invention includes devices of single layer type (anode/light emitting layer/cathode), and the light emitting layer thereof contains a polynuclear complex of the present invention. The layer constitutions of multi-layer type light emitting devices include

(a) anode/hole injection layer/(hole transporting layer)/light emitting layer/cathode
(b) anode/light emitting layer/electron injection layer/(electron transporting layer)/cathode
(c) anode/hole injection layer/(hole transporting layer)/light emitting layer/electron injection layer/(electron transporting layer)/cathode, and the like.

In the above-described constitutions (a) to (c), (hole transporting layer) and (electron transporting layer) represent that each of these layers may be or may not be present at its position.

The anode of a light emitting device of the present invention is an electrode feeding holes to a hole injection layer, hole transporting layer, light emitting layer and the like, and it is effective that the anode has a work function of 4.5 eV or more. As the material of the anode, metals, alloys, metal oxides, electric conductive compounds, mixtures thereof, and the like can be used. Specifically, electric conductive metal oxides such as tin oxide, zinc oxide, indium oxide, indium tin oxide (ITO) and the like, or metals such as gold, silver, chromium, nickel and the like, further, mixtures or laminates of these electric conductive metal oxides and metals, inorganic electric conductive substances such as copper iodide, copper sulfide and the like, organic electric conductive materials such as polyanilines, polythiophenes [PEDOT, and the like], polypyrrole and the like, and laminates of these materials and ITO, and the like are mentioned.

The cathode of a light emitting device of the present invention is an electrode feeding electrons to an electron injection layer, electron transporting layer, light emitting layer and the like, and as the material of the cathode, metals, alloys, metal halides, metal oxides, electric conductive compounds, or mixtures thereof can be used. Specific examples of the material of the cathode include alkali metals (Li, Na, K and the like) and fluorides or oxides thereof, alkaline earth metals (Mg, Ca, Ba, Cs and the like) and fluorides or oxides thereof, gold, silver, lead, aluminum, alloys or mixed metals (sodium-potassium alloy, sodium-potassium mixed metal, lithium-aluminum alloy, lithium-aluminum mixed metal, magnesium-silver alloy, or magnesium-silver mixed metal, and the like), rare earth metals (indium, ytterbium and the like), etc.

The hole injection layer and hole transporting layer of a light emitting device of the present invention may advantageously be layers having any of a function of injecting holes from an anode, a function of transporting holes and a function of blocking electrons injected from a cathode. Known materials can be appropriately selected and used, and specific examples thereof include carbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidine compounds, porphyrin compounds, polysilane compounds, poly(N-vinylcarbazole) derivatives, organic silane derivatives, polynuclear complexes of the present invention and the like, and polymers containing them. Electric conductive polymer oligomers such as aniline copolymers, thiophene oligomers, polythiophenes and the like are also mentioned. The above-described material may be composed of a single component or a composition composed of several components. The above-described hole injection layer and the above-described hole transporting layer may have a single layer structure composed of one or more of the above-described materials, or a multi-layer structure composed of several layers of the same or different compositions.

The electron injection layer and electron transporting layer of a light emitting device of the present invention may advantageously be layers having any of a function of injecting electrons from a cathode, a function of transporting electrons and a function of blocking holes injected from an anode. Known materials can be appropriately selected and used, and specific examples thereof include triazole derivatives, oxazole derivativse, oxadiazole derivatives, imidazole derivatives, fluorenone derivatives, anthraquinodimethane derivatives, anthrone derivatives, diphenylquinone derivatives, thiopyrane dioxide derivatives, carbodiimide derivatives, fluorenylidenemethane derivatives, distyrylpyrazine derivatives, aromatic tetracarboxylic anhydrides such as naphthalene, perylene and the like, phthalocyanine derivatives, various metal complexes typified by metal complexes of 8-quinolinol derivatives and metal complexes having metalphthalocyanine, benzooxazole or benzothiazole as a ligand, and organic silane derivatives, polynuclear complex compounds of the present invention, and the like. The above-described electron injection layer and the above-described electron transporting layer may have a single layer structure composed of one or more of the above-described materials, or a multi-layer structure composed of several layers of the same or different compositions.

As substances constituting the electron injection and transporting layers in a light emitting device of the present invention, insulating or semiconductive inorganic compounds can also be used. When the electron injection and transporting layers are constituted of an insulator or semiconductor, leak of current can be effectively prevented to improve electron injectability. As the insulator, at least one metal compound selected from the group consisting of alkali metal chalcogenides, alkaline earth metal chalcogenides, alkali metal halides and alkaline earth metal halides can be used. Specifically, examples of preferable alkali metal chalcogenides include CaO, BaO ,SrO ,BeO ,BaS and CaSe. As the semiconductor constituting the electron injection and transporting layers, a single member selected from oxides, nitrides, oxide nitrides and the like containing at least one element from Ba ,Ca ,Sr ,Yb ,Al ,Ga ,In ,Li ,Na ,Cd ,Mg ,Si ,Ta Sb and Zn, and a combination composed of two or more of them, are also mentioned.

In the present invention, a reducing dopant may also be added to a boundary region with a thin film in contact with a cathode. The preferable reducing dopant is at least one compound selected from the group consisting of alkali metals, alkaline earth metal oxides, alkaline earth metals, rare earth metals, alkali metal oxides, alkali metal halides, alkaline earth metal oxides, alkaline earth metal halides, rare earth metal oxides or rare earth metal halides, alkali metal complexes, alkaline earth metal complexes and rare earth metal complexes.

The light emitting layer of a light emitting device of the present invention allows injection of holes from an anode or hole injection layer in application of electric field, and has a function of being capable of injecting electrons from a cathode or electron injection layer, a function of moving injected charges (electron and hole) by a force of electric field,

and a function of providing a rebinding place for electrons and holes to cause light emission. The light emitting layer of a light emitting device of the present invention preferably contains at least a polynuclear complex of the present invention, and may also contain a host material containing this polynuclear complex as a guest material. Examples of the above-described host material includes those having a fluorene skeleton, those having a carbazole skeleton, those having a diarylamine skeleton, those having a pyridine skeleton, those having a pyrazine skeleton, those having a triazine skeleton and those having an arylsilane skeleton, and the like. It is preferable that T1 (energy level in the lowest triple excited state) of the above-described host material is larger than that of the guest material, and it is further preferable that the difference thereof is larger than 0.2 eV. The above-described host material may be a low molecular weight compound or a polymer compound. The above-described host material and a light emitting material such as the above-described polynuclear complex or the like are mixed and applied, or subjected to co-vapor deposition or the like, thereby, a light emitting layer containing the above-described light emitting material doped in the above-described host material can be formed.

In the light emitting device of the present invention, the method for forming each of the above-described layers is not particularly restricted and known methods can be used. Specifically mentioned are vacuum vapor deposition methods (resistance heating vapor deposition method, electron beam method and the like), sputtering method, LB method, molecular stacking method, application methods (casting method, spin coat method, bar coat method, blade coat method, roll coat method, gravure printing, screen printing, inkjet method and the like), etc. Of them, film formation by application methods is preferable since the production process can be simplified. In the above-described application methods, the polynuclear complex of the present invention is mixed with a solvent to prepare an application liquid, the application liquid is applied on a given layer (or electrode) and dried, thereby, a film can be formed. The application liquid may contain a rein as a host material and/or binder, and this resin can be dissolved in a solvent, or dispersed in a solvent. As the above-described resin, non-conjugated polymers (for example, polyvinyl carbazole) and conjugated polymers (for example, polyolefin polymer) can be used. More specifically, this resin can be selected depending on its object from polyvinyl chloride, polycarbonate, polystyrene, polymethyl methacrylate, polybutyl methacrylate, polyester, polysulfone, polyphenylene oxide, polybutadiene, poly(N-vinylcarbazole), hydrocarbon resin, ketone resin, phenoxy resin, polyamide, ethylcellulose, vinyl acetate, ABS resin, polyurethane, melamine resin, unsaturated polyester resin, alkyd resin, epoxy resin, silicon resin and the like. The solution may also contain an antioxidant, viscosity regulator and the like as accessory components, depending on the object.

As the solvent for the solution, those which are stable and capable of dissolving or dispersing uniformly components of a thin film can be appropriately selected from known solvents and used. Such solvents include alcohols (methanol, ethanol, isopropyl alcohol, and the like), ketones (acetone, methyl ethyl ketone, and the like), organic chlorine compounds (chloroform, 1,2-dichloroethane and the like), aromatic hydrocarbons (benzene, toluene, xylene, and the like), aliphatic hydrocarbons (normal hexane, cyclohexane and the like), amides (dimethylformamide and the like), sulfoxides (dimethyl sulfoxide and the like), etc. The solvent may be composed of a single component or a mixture of several components.

In the inkjet method, known components can be used for dischargeability of an ink and reproducibility thereof. For example, solvents of high boiling point (anisole, bicyclohexylbenzene and the like) can be used for suppressing evaporation from a nozzle. It is preferable to control the viscosity of a solution to 1 to 100 mPa·s by selecting components.

The preferable thickness of each of organic layers of a light emitting device of the present invention varies depending on the kind of the material and the layer constitution and is not particularly restricted, however, in general, when the film thickness is too thin, defects such as pin holes and the like tend to occur, and in contrast when too thick, high voltage is required to be applied to deteriorate efficiency, thus, usually, the thickness is preferably in the range of several nm to 1 $\mu$m.

Though the use application of the light emitting film and light emitting device of the present invention is not particularly restricted, these can be used for illumination light sources, sign light sources, backlight light sources, display apparatuses, printer heads, and the like. As the display apparatus, known driving technologies, driving circuits and the like can be used and constitutions such as segment type, dot matrix type and the like can be selected. The light emitting film of the present invention can be used also for light wavelength conversion materials and the like, in addition to the above-described use applications.

The present invention will be illustrated further in detail by examples mentioned below, but the present invention is not limited to these examples.

Compounds 1 to 10 in examples have structures as shown below.

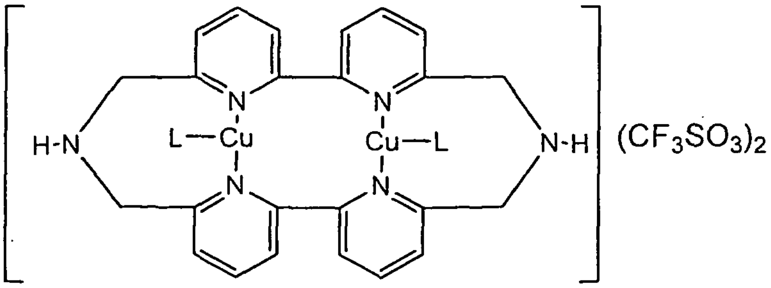

Compound 1: L = acetonitrile
Compound 2: L = triphenylphosphine
Compound 3: L = benzonitrile
Compound 4: L = t-butylpyridine
Compound 5: L = tri(p-methoxyphenyl)phosphine
Compound 6: L = tri(p-fluorophenyl)phosphine
Compound 7: L = tri(p-tolyl)phosphine
Compound 8: L = triphenyl phosphite
Compound 9

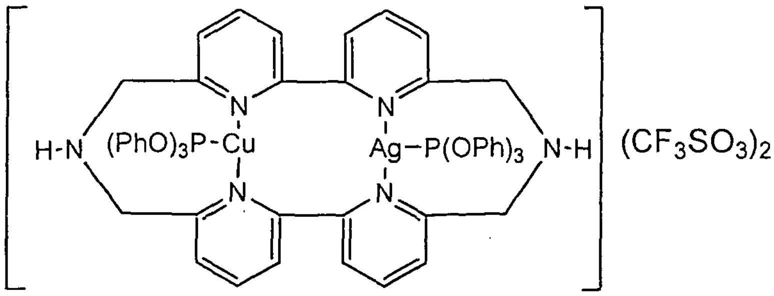

Compound 10

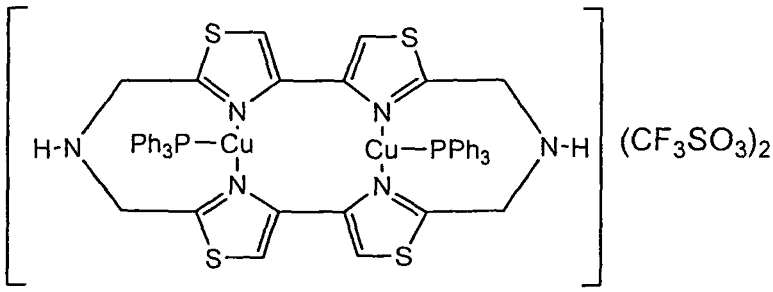

Example 1

<Synthesis of compound 1>

First, a compound (A) was synthesized according to Helv. Chim. Acta., 67, 2264-2269 (1984).

Next, the compound (A) (0.0201 g, 0.051 mmol) was suspended in acetonitrile (4 mL), and tetrakis(acetonitrile) copper(I) trifluoromethanesulfonate (0.0401 g, 0.11 mmol) was added and the mixture was stirred at room temperature for 1 hour. The solvent was removed under a nitrogen flow, and the residue was washed with dichloromethane (2 mL) and dissolved again in acetonitrile (1 mL) and filtrated to obtain a solution which was re-crystallized by a diethyl ether

solvent diffusion method to obtain a compound 1 (0.0442 g, 0.049 mmol, 96%). Element analysis: compound 1 ($C_{30}H_{28}Cu_2F_6N_8O_6S_2$) calculated value C (39.96%), H (3.13%), N (12.43%)/measured value C (40.17%), H (3.19%), N (12.67%)

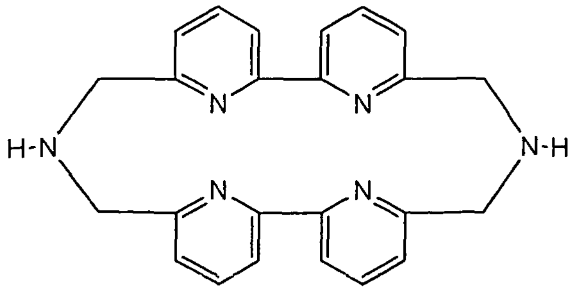

(A)

Example 2

<Synthesis of compound 2>

The compound (A) (0.0101 g, 0.026 mmol) and triphenylphosphine (0.0148 g, 0.056 mmol) were dissolved in chloroform (2 mL), and tetrakis(acetonitrile) copper(I) (trifluoromethanesulfonic acid) salt (0.0205 g, 0.054 mmol) was added. The mixture gradually became a yellow homogenious solution via red-purple color, which was then stirred continuously for 1 hour to find deposition of a milky white product. This product was filtrated and washed with chloroform (2 mL), further washed with diethyl ether (2 mL, three times), and dried to obtain a compound 2.
Yield: 0.0253 g, 0.019 mmol, 73%
Element analysis: chloroform 1 molecule-added product of compound 2 ($C_{63}H_{53}Cl_3Cu_2F_6N_6O_6P_2S_2$) calculated value C (51.70%), H (3.65%), N (5.74%)/measured value C (51.16%), H (3.62%), N (5.99%)

Example 3

A compound 3 was synthesized using benzonitril instead of triphenylphosphine and using dichloromethane instead of chloroform, in the same manner as for the compound 2, and the compound 3 was identified by element analysis (calculated value and measured value of element analysis are shown in Table 1).

Example 4

A compound 4 was synthesized using p-t-butylpyridine instead of triphenylphosphine and using dichloromethane instead of chloroform, in the same manner as for the compound 2, and the compound 4 was identified by element analysis (calculated value and measured value of element analysis are shown in Table 1).

Example 5

A compound 5 was synthesized using tri(p-methoxyphenyl)phosphine instead of triphenylphosphine and using dichloromethane instead of chloroform, in the same manner as for the compound 2, and the compound 5 was identified by element analysis (calculated value and measured value of element analysis are shown in Table 1).

Example 6

A compound 6 was synthesized using tri(p-fluorophenyl)phosphine instead of triphenylphosphine, in the same manner as for the compound 2, and the compound 6 was identified (calculated value and measured value of element analysis are shown in Table 1).

Example 7

A compound 7 was synthesized using tri(p-tolyl)phosphine instead of triphenylphosphine, in the same manner as for the compound 2, and the compound 7 was identified (calculated value and measured value of element analysis

are shown in Table 1).

Example 8

A compound 8 was synthesized using triphenyl phosphite instead of triphenylphosphine, in the same manner as for the compound 2, and the compound 8 was identified (calculated value and measured value of element analysis are shown in Table 1).

Example 9

<Synthesis of compound 9>

The compound (A) (0.0199 g, 0.050 mmol) was dissolved in chloroform (2 mL), and tetrakis(acetonitrile) copper (I) (trifluoromethanesulfonic acid)salt (0.0187 g, 0.050 mmol) was added to provide a dark purple solution. To this was added triphenyl phosphite (0.035 g, 0.11 mmol) to observe scarce change. Further, when trifluoromethanesulfonic acid silver(I) salt (0.0128 g, 0.050 mmol) was added, the reaction mixture became a heterogenious suspension of nearly white color. The suspension was stirred for 2 hours, then, 8 mL of diethyl ether was added to cause precipitation of a product, this product was filtrated, and further, washed with diethyl ether (2 mL, three times) and dried to obtain a compound 9. yield: 0.0485 g. The compound 9 was identified by element analysis (calculated value and measured value of element analysis are shown in Table 1).

Example 10

First, a compound(B) was synthesized. Under purging with argon, 0.97 g (5.6 mmol) of tosyl amide was dissolved in 200 ml of dehydrated N,N-dimethylformamide, the solution was cooled to 10°C and 0.23 g (5.6 mmol) of 60% sodium hydride was added while stirring. This mixture was heated up to room temperature, and stirred for 30 minutes, then, cooled to -65°C and 1.0 g (2.8 mmol) of 4,4'-bis(bromomethyl)-2,2'-bithiazole (synthesized according to Helv. Chim. Acta., 75, 1221-1236 (1992)) was gradually added. Thereafter, the mixture was heated up to room temperature, further heated at 78 to 80°C, and reacted for 20 hours. After completion of the reaction, the reaction mixture was put into 300 ml of ice water, and the precipitate was filtrated and washed with water, ethanol and acetone, and dried. The product was purified by silica gel column (developing solvent: chloroform/methanol = 30/1) to obtain 0.29 g of the following compound (B').

Subsequently, 0.28 g (0.39 mmol) of the compound (B') was dissolved in 1.5 ml of 98% sulfuric acid, and reacted at 110°C for 2 hours. The reaction mixture was put into 20 ml of ice water, and rendered alkaline with a 10% sodium hydroxide aqueous solution, then, the deposited precipitate was filtrated, washed with water and dried. The product was purified by silica gel column (developing solvent: chloroform/methanol = 5/1) to obtain 0.10 g (0.23 mmol) of a compound (B). In $^1$H-NMR ($CDCl_3$), peaks were observed at 1.56 ppm (N-H: overlapped with $H_2O$), 4.06 ppm (-$CH_2$-) and 7.25 ppm (C-H of thiazole ring: overlapped with $CHCl_3$).

Next, a compound 10 was synthesized using the compound (B) instead of the compound (A), in the same manner as for the compound 2, and the compound 10 was identified by element analysis: compound 10 ($C_{54}H_{44}N_6Cu_2F_6O_6P_2S_2$) calculated value C (47.40%), H (3.24%), N (6.14%)/measured value C (46.84%), H (3.48%), N (6.26%)

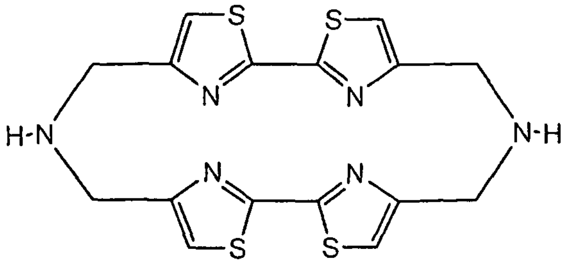

(B)

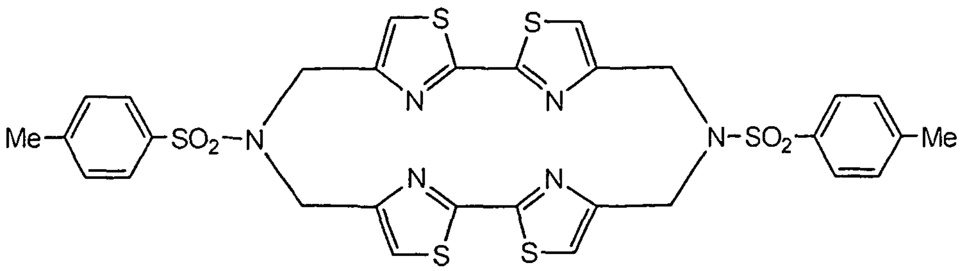

(B')

[Light emitting property test]

For each of the compounds 1 to 9, fine particles of the compound were dispersed in a 0.8 wt% PMMA (polymethyl methacrylate)/toluene solution to make a dispersion (the amount of the compound was 2.0 wt% with respect to the whole dispersion). This was dried and fixed on a quartz plate to prepare a sample. The fluorescent spectrum of this thin film was measured at an excitation wavelength of 350 nm using a fluorescent spectrophotometer (Fluorolog manufactured by JOBINYVON-SPEX). For obtaining the relative light emission intensity on the thin film, a light emission spectrum plotted against wavenumber utilizing the intensity of the Raman line of water as a standard was integrated in the spectrum measurement range, and allotted the absorbances at excitation wavelengths measured using a spectrophotometer (Cary5E, manufactured by Varian). The relative values of the light emission intensities of the compounds 2 to 8 were calculated using the value of the compound 1 as a standard (=1) (relative intensities are described in Table 1).

Table 1 shows light emission maximum wavelengths and relative intensities together with element analyses (measured value is described in upper column, and calculated value is described in parentheses in lower column), for compounds 1 to 9.

[Table 1]

| Compound | Molecular formula | C/% | H/% | N/% | Emission maximum (nm) | Relative intensity |
|---|---|---|---|---|---|---|
| 1 | $C_{30}H_{28}Cu_2F_6N_8O_6S_2$ | 40.17<br>(39.96) | 3.19<br>(3.13) | 12.67<br>(12.43) | 527 | 1 |
| 2 | $C_{63}H_{53}Cl_3Cu_2F_6N_6O_6P_2S_2$ | 51.16<br>(51.70) | 3.62<br>(3.65) | 5.99<br>(5.74) | 496 | 7.7 |
| 3 | $C_{40.5}H_{33}ClCu_2F_6N_8O_6S_2$ ($CH_2Cl_2$ 0.5 molecule added) | 45.52<br>(45.53) | 3.22<br>(3.11) | 10.53<br>(10.49) | 513 | 2.4 |
| 4 | $C_{45}H_{50}Cl_2Cu_2F_6N_8O_6S_2$ ($CH_2Cl_2$ 1 molecule added) | 45.73<br>(46.00) | 4.50<br>(4.29) | 9.46<br>(9.54) | 549 | 0.47 |
| 5 | $C_{70}H_{68}N_6Cu_2F_6O_{12}P_2S_2Cl_4$ ($CH_2Cl_2$ 2 molecule added) | 49.57<br>(49.62) | 4.06<br>(4.05) | 5.06<br>(4.96) | 505 | 11 |
| 6 | $C_{62.5}H_{46.5}Cl_{1.5}Cu_2F_{12}N_6O_6P_2S_2$ ($CHCl_3$ 0.5 molecule added) | 49.49<br>(49.65) | 3.01<br>(3.10) | 5.35<br>(5.56) | 512 | 14 |
| 7 | $C_{69}H_{65}Cl_3Cu_2F_6N_6O_6P_2S_2$ ($CHCl_3$ 1 molecule added) | 53.47<br>(53.54) | 4.33<br>(4.23) | 5.46<br>(5.43) | 501 | 9.1 |
| 8 | $C_{62.5}H_{52.5}Cl_{1.5}Cu_2F_6N_6O_{12}P_2S_2$ ($CHCl_3$ 0.5 molecule added) | 49.28<br>(50.05) | 3.55<br>(3.53) | 5.84<br>(5.60) | 494 | 4.9 |
| 9 | $C_{63.5}H_{53.5}Cl_{4.5}N_6AgCuF_6O_{12}P_2S_2$ ($CHCl_3$ 1.5 molecule added) | 46.23<br>(45.84) | 3.33<br>(3.24) | 5.19<br>(5.06) | 498 | - |

Regarding the element analysis, the measured value is described in the upper column, and the calculated value is described in parentheses in the lower column.

[Light emission life property]

For the compounds 1 and 2, the light emission life was measured by a frequency modulation method. Analysis thereof was performed according to a theoretical formula shown in Anal. Chem. 68, 9-17 (1996).

<Measurement of light emission life of compound 1>

Given that light emission has two components (phosphorescence emission and fluorescence emission), m is modulation, $\tau_1$ is light emission life of component 1, $\tau_2$ is light emission life of component 2, f is proportion of component having light emission life $\tau_1$ ($0 \leq f \leq 1$, proportion of component having $\tau_2$ is 1-f), and $\omega$ is $2\pi \times$frequency (namely, angular frequency), Fig. 1 shows calculation values plotted based on the theoretical formula of m represented by

$$m = [\{f\omega\tau_1/(1+\omega^2\tau_1{}^2)+(1-f)\omega\tau_2/(1+\omega^2\tau_2{}^2)\}^2+\{f/(1+\omega^2\tau_1{}^2)+(1-f)/(1+\omega^2\tau_2{}^2)\}^2]^{1/2}$$

wherein $\tau_1$=0.20(1)$\mu$s, $\tau$2=0.002(7)$\mu$s and f=0.48(1), which were determined by a least square method from the measured values of m.

From this result, a component of 0.20(1)$\mu$s contained in an amount of 48(1)% is ascribable to triplet light emission, and a component of 0.002(7)$\mu$s contained in an amount of 52(1)% is ascribable to singlet light emission.

<Measurement of light emission life of compound 2>

Given that light emission has a single component, m is modulation, $\tau$ is light emission life, and $\omega$ is $2\pi \times$frequency (namely, angular frequency), Fig. 2 shows calculation values plotted based on the theoretical formula of m represented by

$$m = (1+\omega^2\tau^2)^{-1/2}$$

wherein $\tau$=5.04(7)$\mu$s, which was determined by a least square method from the measured values of m.

The single light emission life of 5.04(7)$\mu$s is ascribable to triplet light emission.

Comparative Example 1

A compound 11 which was a mononuclear copper(I) complex using the compound A as a ligand was synthesized and compared.

Compound 11

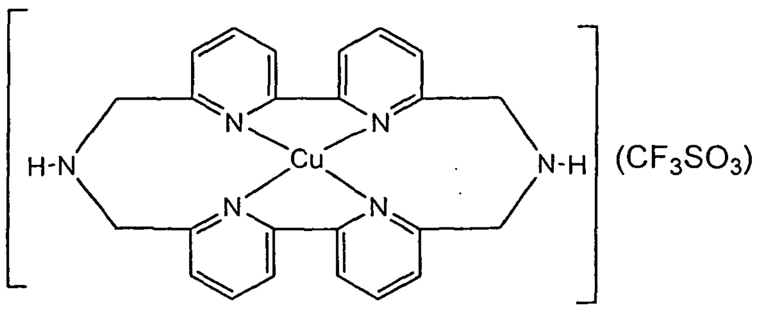

The compound (A) (0.0209 g, 0.053 mmol) was suspended in acetonitrile (2 mL), and tetrakis(acetonitrile) copper(I) trifluoromethanesulfonic acid salt (0.0177 g, 0.047 mmol) dissolved in acetonitrile (3 mL) was added and stirred

at room temperature for 1 hour. The solvent was concentrated to about half under a nitrogen flow, and filtration was performed to obtain a solution which was re-crystallized by a diethyl ether solvent diffusion method to obtain a compound 11 (13.2 mg, 0.022 mmol, 46%). The compound 11 was identified by element analysis.

Element analysis: compound 11 ($C_{25}H_{22}CuF_3N_6O_3S$) calculated value C (49.46%), H (3.65%), N (13.84%)/ measured value C (49.42%), H (3.62%), N (13.67%).

The compound was subjected to the light emission property test in the same manner as for the compounds 1 to 9, to observe light emission of a relative intensity of 0.025 with respect to the compound 1, at an emission maximum of 507 nm.

According to Examples 1 to 9 and Comparative Example 1, novel polynuclear complexes of the present invention containing a macrocyclic ligand having hetero rings connected in cyclic form showed a phosphorescence light emission property having light emission intensity which is significantly larger as compared with mononuclear complexes.

INDUSTRIAL APPLICABILITY

The polynuclear complex of the present invention forms a polynuclear structure relatively easily, and has functions such as a light emission property, catalytic activity and the like, thus, is useful particularly as a light emission material.

## Claims

1. A polynuclear complex having two or more metal atoms and/or metal ions per one ligand of the general formula (I):

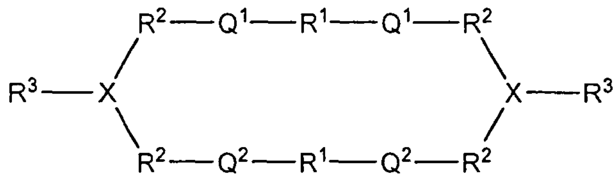

(I)

(wherein, $Q^1$ and $Q^2$ represent each independently a divalent heterocyclic group optionally having a substituent, two $Q^1$s may bond directly or indirectly to form a ring , and two $Q^2$s may bond directly or indirectly to form a ring. $R^1$ and $R^2$ represent each independently a direct bond or an optionally substituted divalent hydrocarbon group, X represents a nitrogen atom or phosphorus atom, $R^3$ represents a monovalent organic group containing an atom selected from a nitrogen atom, oxygen atom, phosphorus atom and sulfur atom, or a hydrogen atom or hydrocarbon group optionally having a substituent, two $R^3$s may bond directly or indirectly to form a ring, and a plurality of $Q^1$s, $Q^2$s, $R^1$s, $R^2$s, $R^3$s and Xs may be mutually the same or different, respectively.).

2. The polynuclear complex according to Claim 1, wherein the ligand of the above-described general formula (I) is a ligand of the following general formula (II):

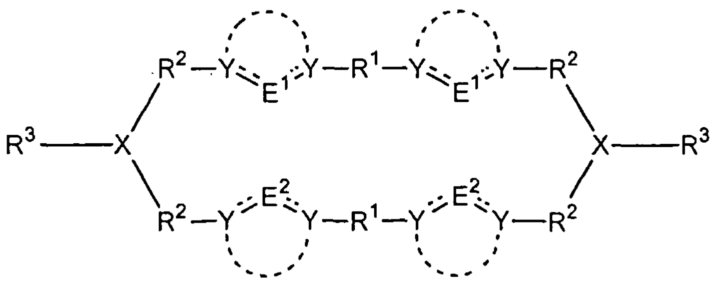

(II)

(wherein, $R^1$, $R^2$, $R^3$ and X represent each independently the same meaning as described above. $E^1$ and $E^2$ represent each independently a nitrogen atom, phosphorus atom, oxygen atom or sulfur atom, Y represents a carbon atom or nitrogen atom, and a plurality of $E^1$s, $E^2$s, $R^1$s, $R^2$s, $R^3$s, Xs and Ys may be mutually the same or

different, respectively. Two $R^3$ may bond directly or indirectly to form a ring, a ring represented by

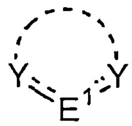

optionally has a substituent,
two rings represented by

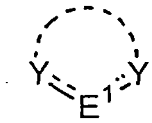

may form a ring directly or together with the substituent on the ring, and a ring represented by

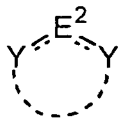

optionally has a substituent,
two rings represented by

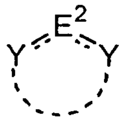

may form a ring directly or together with the substituent on the ring.).

**3.** The polynuclear complex according to Claim 2, wherein the ligand of the above-described general formula (II) is a ligand of the following general formula (III):

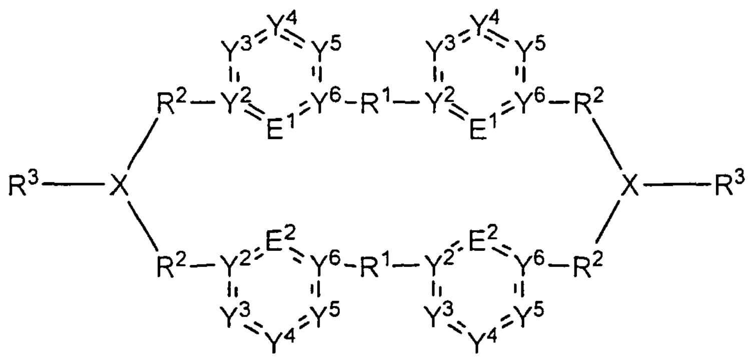

(III)

(wherein, $E^1$, $E^2$, X, $R^1$, $R^2$ and $R^3$ represent each independently the same meaning as described above. $Y^2$ and $Y^6$ represent each independently a carbon atom or nitrogen atom, $Y^3$ and $Y^5$ represent each independently C(H), nitrogen atom, N(H), oxygen atom or sulfur atom, $Y^4$ represents a direct bond, C(H), nitrogen atom, oxygen atom or sulfur atom, and a plurality of $E^1$s, $E^2$s, $R^1$s, $R^2$s, $R^3$s, Xs and $Y^3$s to $Y^5$s may be mutually the same or different, respectively. Two $R^3$ may bond directly or indirectly to form a ring,

a ring represented by

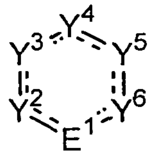

optionally has a substituent,
substituents on two rings represented by

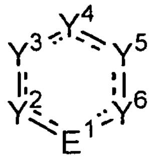

may together form a ring,
and a ring represented by

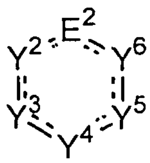

optionally has a substituent,
substituents on two rings represented by

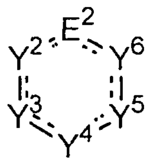

may together form a ring.).

**4.** The polynuclear complex according to Claim 3, wherein the ligand of the above-described general formula (III) is a ligand of the following general formula (IV) or (V):

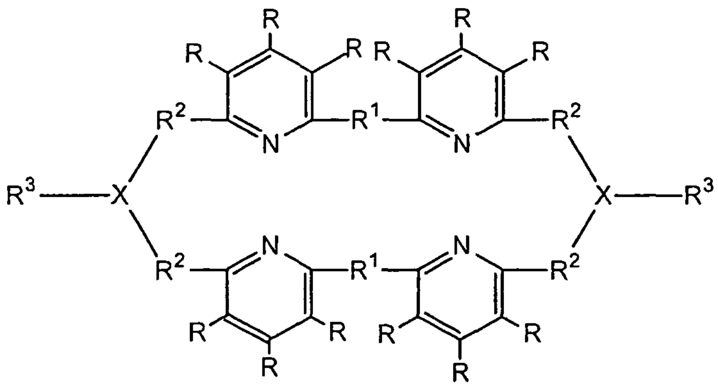

(IV)

(wherein, X, $R^1$, $R^2$ and $R^3$ represent each independently the same meaning as described above. R represents a hydrogen atom or substituent, and two Rs together may form a ring.)

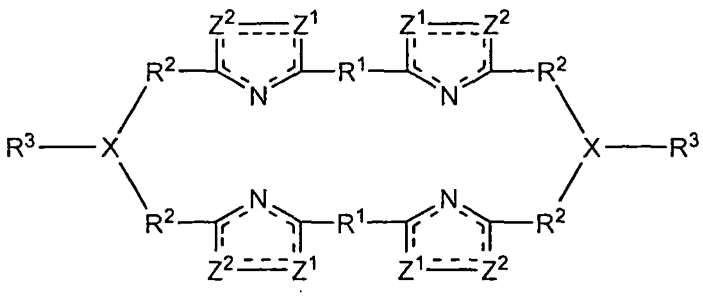

(V)

(wherein, X, $R^1$, $R^2$ and $R^3$ represent each independently the same meaning as described above. Either $Z^1$ or $Z^2$ is C(R') and another is an oxygen atom, sulfur atom or N(R''), $Z^1$s may be mutually the same or different and $Z^2$s may be mutually the same or different. R' and R'' represent each independently a hydrogen atom or substituent, and two R's or R''s together may form a ring.).

**5.** The polynuclear complex according to any one of Claims 1 to 4, wherein the number of d electrons of the metal ion and/or metal atom is an even number.

**6.** The polynuclear complex according to Claim 5, wherein the number of d electrons of the metal ion and/or metal atom is 6, 8 or 10.

**7.** The polynuclear complex according to Claim 6, wherein the number of d electrons of the metal ion and/or metal atom is 10.

**8.** The polynuclear complex according to Claim 7, wherein the metal ion is a copper(I) ion or silver(I) ion.

**9.** The polynuclear complex according to any one of Claims 1 to 8, wherein the complex has a phosphorescence emitting property.

**10.** A luminous film comprising the polynuclear complex as described in any one of Claims 1 to 9.

**11.** A light emitting device comprising the polynuclear complex as described in any one of Claims 1 to 9.

**12.** A ligand compound of the following general formula (VI)

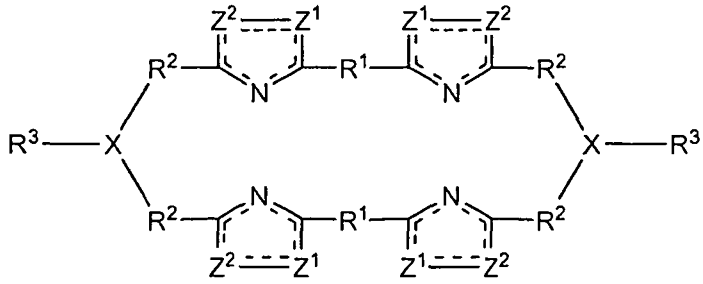

(VI)

(wherein, X, $R^1$, $R^2$ and $R^3$ represent each independently the same meaning as described above. Either $Z^1$ or $Z^2$ is C(R'), and another is an oxygen atom, sulfur atom or N(R''), $Z^1$s may be mutually the same or different and $Z^2$s may be mutually the same or different. R' and R'' represent each independently a hydrogen atom or substituent.).

Fig. 1

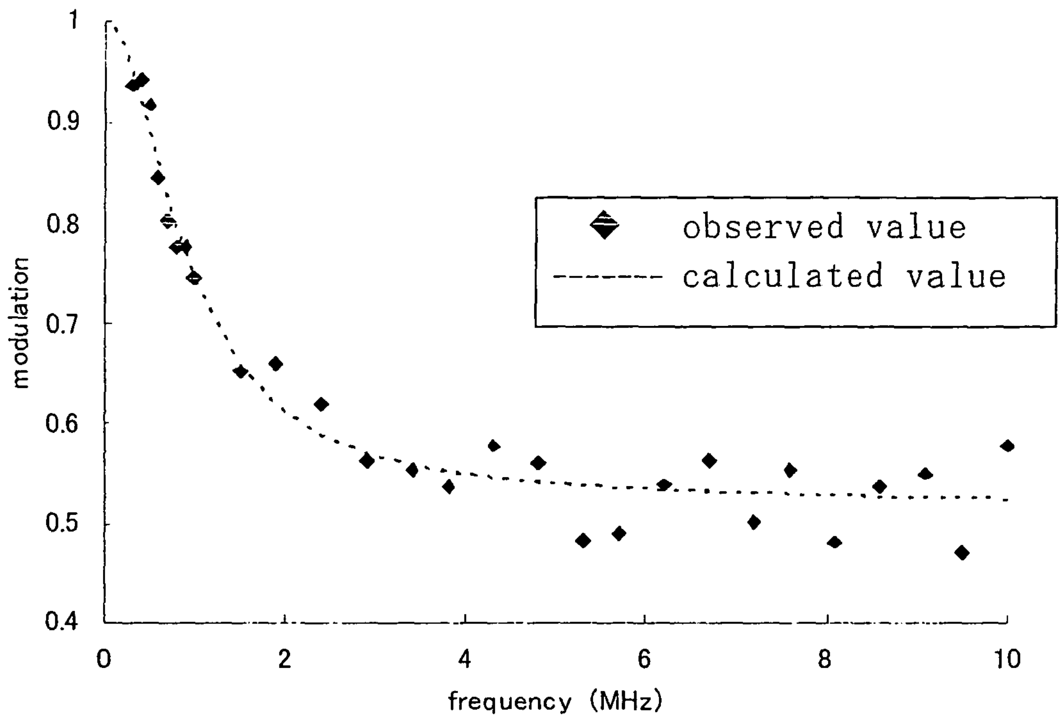
observed value
calculated value
modulation
frequency (MHz)
1
0.9
0.8
0.7
0.6
0.5
0.4
0
2
4
6
8
10

Fig. 2

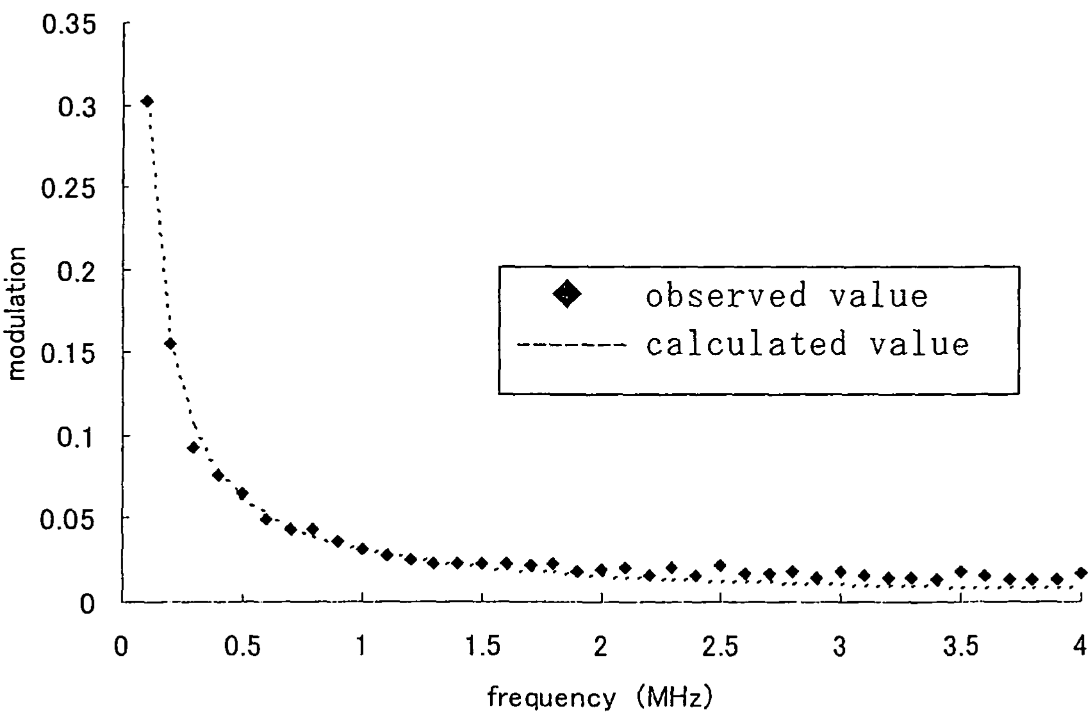
observed value
calculated value
modulation
frequency (MHz)
0.35
0.3
0.25
0.2
0.15
0.1
0.05
0
0
0.5
1
1.5
2
2.5
3
3.5
4

**INTERNATIONAL SEARCH REPORT**

International application No.
PCT/JP2007/073259

A. CLASSIFICATION OF SUBJECT MATTER
*C07D471/22*(2006.01)i, *C09K11/06*(2006.01)i, *H01L51/50*(2006.01)i, *C07F1/08*(2006.01)n

According to International Patent Classification (IPC) or to both national classification and IPC

B. FIELDS SEARCHED

Minimum documentation searched (classification system followed by classification symbols)
C07D471/22, C09K11/06, H01L51/50, C07F1/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), CAOLD(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | LEHN, Jean Marie et al., Synthesis of macrobicyclic cryptates incorporating bithiazole, bisimidazole and bipyrimidine binding subunits, Tetrahedron Letters, 1989, Vol.30, No.17, pp.2209-2212, full text, particularly, compounds 1a, 1b | 12 |
| A | JP 6-510296 A (CIS Bio International), 17 November, 1994 (17.11.94), Full text & WO 93/05049 A1 & EP 601113 A1 & US 5457184 A & FR 2680787 A1 & AU 9225680 A | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 January, 2008 (09.01.08) | 29 January, 2008 (29.01.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/073259

C (Continuation). DOCUMENTS CONSIDERED TO BE RELEVANT

| Category* | Citation of document, with indication, where appropriate, of the relevant passages | Relevant to claim No. |
|---|---|---|
| A | ZHOU, Hong et al., Study on the crystal structure of a macrocycle and tyrosinase activity of its dinuclear copper complexes, Chinese Journal of Chemistry, 2005, Vol.23, No.7, pp.835-842 | 1-12 |
| A | STANKOVA, Ivanka G. et al., Synthesis of novel imidazole, thiazole, oxazole substituted peptides, cyclotetrapeptide and their antibacterial activity in vitro, Peptides 1998, Proceedings of the European Peptide Symposium, 25th, Budapest, Aug. 30-Sept. 4, 1998, 1999, pp.248-249 | 1-12 |
| A | RODRIGUEZ-UBIS, Juan Carlos et al., Photoactive Cryptands. Synthesis of the sodium cryptates of macrobicyclic ligands containing bipyridine and phenanthroline groups, Helvetica Chimica Acta, 1984, Vol.67, No.8, pp.2264-2269 | 1-12 |

Form PCT/ISA/210 (continuation of second sheet) (April 2007)

**INTERNATIONAL SEARCH REPORT**

International application No.
PCT/JP2007/073259

<Subject of search>

Claims 1-12 relate to compounds represented by the general formulae (I)-(VI), and the compounds represented by the general formulae (I)-(VI) include great many compounds having various combinations with respect to size and properties. However, among these compounds, those which are supported by the description in the meaning within PCT Article 6 and disclosed in the meaning within PCT Article 5 are limited to an extremely small part of the compounds as claimed.

Such being the case, the search was made on the parts supported by and disclosed in the description, namely the compounds disclosed in working examples.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Chem. Rev.,* 1996, vol. 96, 759-833
- *Helv. Chim. Acta.,* 1984, vol. 67, 2264-2269
- *Helv. Chim. Acta.,* 1992, vol. 75, 1221-1236
- *Anal. Chem.,* 1996, vol. 68, 9-17